(19) Europäisches Patentamt
European Patent Office
Office européen des brevets

(11) EP 3 684 477 B1

(12) # FASCICULE DE BREVET EUROPEEN

(45) Date de publication et mention de la délivrance du brevet:
**02.03.2022 Bulletin 2022/09**

(21) Numéro de dépôt: **18701268.7**

(22) Date de dépôt: **09.01.2018**

(51) Classification Internationale des Brevets (IPC):
***A61Q 19/00*** (2006.01) ***A61K 8/92*** (2006.01)
***A61K 8/34*** (2006.01) ***A61K 8/9789*** (2017.01)

(52) Classification Coopérative des Brevets (CPC):
**A61Q 19/008; A61K 8/347; A61K 8/922; A61K 8/9789**

(86) Numéro de dépôt international:
**PCT/EP2018/050482**

(87) Numéro de publication internationale:
**WO 2019/137603 (18.07.2019 Gazette 2019/29)**

(54) **COMPOSITIONS UTILES POUR LE TRAITEMENT COSMÉTIQUE DES PEAUX GRASSES**

KOSMETISCHE ZUSAMMENSETZUNGEN ZUR BEHANDLUNG FETTIGER HAUT

COSMETIC COMPOSITIONS FOR TREATING OILY SKINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**29.07.2020 Bulletin 2020/31**

(73) Titulaire: **Tomcat International Limited**
**London W1W 8DQ (GB)**

(72) Inventeurs:
- **THOMAS, Mathilde**
  **75007 PARIS (FR)**
- **THOMAS, Bertrand**
  **75007 PARIS (FR)**

(74) Mandataire: **Germain Maureau**
**12, rue Boileau**
**69006 Lyon (FR)**

(56) Documents cités:
**FR-A1- 2 715 848**
**FR-A1- 2 770 228**
**FR-A1- 2 836 377**
**GB-A- 2 469 289**
**US-A1- 2004 234 480**

- **DATABASE WPI Week 201509 Thomson Scientific, London, GB; AN 2015-05076R XP002784662, & CN 104 146 915 A (SHINAN BRANCH QINGDAO LANTU CULTURE COMM) 19 novembre 2014 (2014-11-19)**
- **DATABASE GNPD [Online] MINTEL; 1 octobre 2013 (2013-10-01), "Step 03 Medicated Acne care Essence", XP002784663, Database accession no. 2201538**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne l'amélioration de l'apparence des peaux grasses ou à tendance grasse et des signes cutanés qui leur sont associés. Elle concerne ainsi de nouvelles associations d'actifs utilisables à cet effet et ayant une activité synergique, et des compositions les contenant. L'invention concerne encore l'utilisation non thérapeutique cosmétique de telles associations et compositions, pour traiter les peaux grasses ou à tendance grasse et des procédés de traitement non thérapeutique cosmétique de telles peaux.

La peau sécrète naturellement une substance grasse, le sébum, qui constitue normalement un agent hydratant de l'épiderme et peut être impliqué dans l'homéostasie de l'épiderme. Il contribue à sa protection contre les agressions extérieures, par des activités antioxydantes ou antimicrobiennes notamment.

Le sébum est le produit naturel de la glande sébacée qui constitue une annexe de l'unité pilosébacée. Il s'agit essentiellement d'un mélange plus ou moins complexe de lipides. Une peau grasse ou hyperséborrhéique est caractérisée, notamment, par une sécrétion et une excrétion exagérées de sébum. Classiquement, un taux de sébum supérieur à 220 $\mu g/cm^2$ mesuré au niveau du front est considéré comme caractéristique d'une telle peau grasse.

La peau grasse est un problème cosmétique fréquent qui apparaît lorsque les glandes sébacées produisent un excès de sébum (séborrhée), donnant à la peau une apparence grasse et luisante. Ce phénomène est également associé à une augmentation de la taille des pores, un grain de peau grossier et irrégulier, des comédons, considérés comme des imperfections cutanées et des défauts esthétiques.

Les glandes sébacées qui produisent le sébum sont localisées dans le derme réticulaire où elles sont associées aux poils, l'ensemble formant les unités pilo-sébacées. La densité des glandes pilo-sébacées varie de manière importante selon leur localisation au niveau du corps. Elles sont abondantes au niveau du visage, notamment au niveau du front, du nez et du menton (Zone T), ainsi qu'au niveau du cuir chevelu. Ainsi ces zones sont aussi les plus fréquemment associées au problème de peau grasse.

De nombreux facteurs peuvent contribuer à l'augmentation de la séborrhée : facteurs hormonaux, génétiques, l'âge, le sexe, l'origine ethnique, les variations climatiques, la pollution, le stress, la fatigue, le microbiome par exemple.

Les peaux grasses sont généralement des peaux saines, qui résistent bien par exemple aux signes du vieillissement ; elles n'évoluent pas vers d'autres troubles. Toutefois, leur aspect jugé inesthétique et les imperfections cutanées qui peuvent être présentes, conduisent à rechercher des solutions pour les traiter et lutter contre les signes et les désordres qui leur sont associés.

De telles peaux grasses ou à tendance grasse, et leurs défauts esthétiques ou imperfections cutanées, peuvent être associés, outre l'excès de sébum, au développement de germes *Propionibacterium acnes*. Le germe *Propionibacterium acnes* (P. *acnes*) fait partie des bactéries anaérobies à Gram positif qui préfèrent pousser dans des environnements pauvres en oxygène, comme au fond d'un follicule pileux ou d'un canal sébacé obstrué.

*Propionibacterium acnes* contribuerait au problème des peaux grasses à imperfections, non-acnéiques. En effet chez des sujets en bonne santé, sans historique de désordres cutanés, une augmentation de la quantité de sébum a été corrélée à une augmentation de la prévalence de *Propionibacterium* et une diminution de la diversité microbienne. De même, une corrélation a été montrée entre la prévalence de *Propionibacterium acnes* et la quantité de lipides cutanés produits chez des sujets en bonne santé sans historique de désordres cutanés. Enfin il a été montré que *Propionibacterium acnes* peut contribuer directement à une augmentation de la lipogenèse et donc à une augmentation de la production de sébum.

Par ailleurs l'acné est une pathologie inflammatoire fréquente affectant le follicule pilosébacé, dans laquelle *Propionibacterium acnes* est impliqué. Elle est caractérisée par la présence de lésions rétentionelles (microkystes) et/ou inflammatoires (papules, pustules, nodules). L'étiopathologie de l'acné est multifactorielle et reste en partie à élucider. Cependant la quantité et la qualité du sébum jouent un rôle clé dans la prolifération de la bactérie *Propionibacterium acnes* qui contribue à l'état inflammatoire cutané.

FR 3 006890 propose l'utilisation cosmétique de dermicidine pour la prévention ou le traitement des peaux grasses.

L'utilisation d'huiles essentielles a aussi été proposée pour lutter contre les signes cutanés associés aux peaux grasses. Par exemple FR 3 004937 concerne l'utilisation cosmétique d'une huile essentielle d'*Origanum majorana* ; FR 3 004939 décrit l'utilisation d'huile essentielle de *Satureja montana* pour le traitement cosmétique des peaux grasses.

CN104146915 décrit un article cosmétique pour hydrater la peau et présentant une activité anti-acnéique, contenant un mélange d'huiles essentielles.

Toutefois, il existe toujours un besoin de disposer de nouveaux actifs ayant une efficacité cosmétique améliorée sur la production de sébum et les signes associés à la production accrue de sébum.

De manière inattendue, il a maintenant été trouvé qu'une association de polyphénols et d'un mélange d'huiles essentielles permet ainsi de lutter efficacement contre les défauts esthétiques associés aux peaux grasses ou à tendance grasse.

C'est pourquoi la présente invention a pour objet l'utilisation non thérapeutique cosmétique d'une association de

(i) au moins un polyphénol choisi parmi les monomères et oligomères de flavonoïdes extraits de pépins de raisin, leurs dérivés et leurs mélanges, et

(ii) d'huiles essentielles de menthe poivrée, de géranium, de mélisse officinale, de lemongrass, de romarin et de lavandin,

pour traiter et/ou prévenir les peaux grasses ou à tendance grasse et/ou les désordres esthétiques cutanés qui leur sont associés.

L'invention a également pour objet un procédé de traitement non thérapeutique cosmétique des peaux grasses ou à tendance grasse et/ou des désordres esthétiques cutanés associés aux peaux grasses ou à tendance grasse, qui comprend l'application topique sur la peau d'une association de (i) au moins undit polyphénol et (ii) un mélange d'huiles essentielles menthe poivrée, de géranium, de mélisse officinale, de lemongrass, de romarin et de lavandin, ou d'une composition cosmétique contenant ladite association.

L'invention a encore pour objet une composition, en particulier une composition cosmétique, contenant une telle association dans un milieu physiologiquement acceptable ; la composition est de préférence adaptée à une application topique sur la peau. En particulier, la composition cosmétique peut contenir une association de

(i) au moins un polyphénol choisi parmi les monomères et oligomères de flavonoïdes extraits de pépins de raisin, leurs dérivés et leurs mélanges, et

(ii) d'huiles essentielles de menthe poivrée, de géranium, de mélisse officinale, de lemongrass, de romarin et de lavandin,

dans un milieu physiologiquement acceptable.

Les polyphénols adaptés à la mise en oeuvre de l'invention sont choisis parmi les monomères et oligomères de f|avonoïdes d'extrait de vigne.

Ils peuvent être obtenus à partir de la plante *Vitis vinifera.*

Ces polyphénols peuvent être sous forme native ou stabilisée.

Les polyphénols convenant à l'invention sont des flavonoides, tels que les monomères et oligomères de flavan-3-ols, en particulier catéchine, epicatéchine, gallocatéchines, (par proanthocyanidine ou OPC, les anthocyanes ou les flavanones ; on peut citer encore les épigallocatéchines exemple épigallocatéchine gallate) et oligomères deflavonols, dihydroflavonols, flavones et isoflavones.

Avantageusement, les polyphénols ou les dérivés de polyphénols comprennent des catéchines, et/ou les oligomères de catéchines, ou des dérivés de ces composés.

Les oligomères de catéchines comprennent de préférence de 2 à 6 unités catéchiques (catéchine ou épicatéchine) et sont aussi appelés OPC.

Les unités monomères des flavonoïdes répondent à la structure (I) suivante

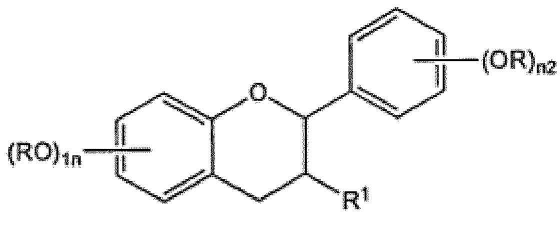

(I)

dans laquelle

- R1 représente un groupe -OR2, un atome d'hydrogène, ou un substituant R3 ; R2 et R3 identiques ou différents, étant tels que défini ci-dessous pour R4
- au moins la majorité des substituants R représentent un groupe -COR4, R4 étant un radical alkyle d'au moins deux atomes de carbone, linéaire ou ramifié, saturé ou insaturé, un radical aryle, arylalkyle ou arylalkylène,
- le ou les autres substituants R qui ne représentent pas un groupe -COR4 étant un atome d'hydrogène, ou un groupe alkyle, et
- n1 et n2, identiques ou différents l'un de l'autre, sont des nombres de 1 à 3, correspondant au nombre de substitutions sur un cycle,

les motifs monomères étant reliés dans les oligomères et les polymères par des liaisons carbone-carbone et /ou un pont

éther entre les unités.

Dans les oligomères et polymères de l'invention, les liaisons entre les atomes de carbone des motifs successifs sont situées entre le C-4 d'un motif et le C-6 ou le C-8 d'un autre motif.

Dans d'autres oligomères et polymères, au moins 2 motifs sont reliés en outre par un pont oxygène.

Dans une composition préférée, les polyphénols sont des extraits flavonoïdiques de pépins de raisins (OPC), renfermant des monomères, des oligomères et/ou des polymères d'unités répondant à la formule (I).

Par dérivés des polyphénols on entend plus particulièrement des molécules stabilisées dans lesquelles tout ou partie des groupements hydroxyles sont éthérifiés ou estérifiés. Selon un mode de réalisation préféré, l'estérification est formée avec un acide gras saturé ou insaturé. L'acide gras peut notamment être choisi parmi l'acide butyrique, valérique, hexanique, sorbique, laurique, palmitique, stéarique, oléique, linoléique, linolénique, alpha-linolénique, arachidonique, écosapentaénoïque et docosahexaénoïque.

Selon un mode de réalisation particulier, l'ensemble des groupements hydroxyles du polyphénol sont estérifiées ou éthérifiées, le dérivé de polyphénol ne présente donc plus de fonction OH libre. De tels éthers ou esters peuvent notamment être préparés par le procédé décrit dans la demande WO 2011/128714.

L'association selon l'invention comprend en particulier au moins un polyphénol choisi parmi les monomères et oligomères de flavan-3-ols, tels que les monomères et oligomères de proanthocyanidines (OPC), leurs dérivés et leurs mélanges en toutes proportions.

Avantageusement, dans une composition selon l'invention, les polyphénols mentionnés en (i) comprennent des OPC extraits de pépin de raisin. En particulier l'association ou la composition comprend des dérivés de polyphénols qui sont des esters de polyphénols.

Selon un mode de réalisation particulier, les polyphénols sont présents dans l'association ou la composition sous forme d'extraits de pépins de raisin, éventuellement stabilisés pour obtenir des dérivés de polyphénols dans lesdits extraits.

De tels polyphénols comprennent généralement au moins 20% (en poids, par rapport au poids de polyphénols totaux) de monomères et/ou dimères de catéchines ; par exemple, la teneur en dimères de proanthocyanidine est supérieure ou égale à 20 % (en poids, par rapport au poids de polyphénols totaux), et/ou la teneur en [catéchine et épicatéchine] est supérieure ou égale à 20 % (en poids, par rapport au poids de polyphénols totaux).

Selon un mode de réalisation avantageux, au moins un polyphénol est choisi parmi les monomères et oligomères de proanthocyanidines extraits de pépins de raisin, sous forme native ou sous forme estérifiée et leurs mélanges.

L'autre constituant de l'association selon l'invention est un mélange d'huiles essentielles, à savoir l'huile essentielle de menthe poivrée (*Mentha piperita*), l'huile essentielle de géranium (*Pelargonium graveolens*), l'huile essentielle de mélisse officinale (Melissa *officinalis*), l'huile essentielle de lemongrass (*Cymbopogon flexuosus*), l'huile essentielle de romarin (*Rosmarinus officinalis*), et l'huile essentielle de lavandin (*Lavandula hybrida*).

Les huiles essentielles sont des produits obtenus à partir de matières premières d'origine végétale (feuilles, tiges, fleurs ou plante entière par exemple).

Ces huiles essentielles peuvent être obtenues selon différents procédés, tels qu'entraînement à la vapeur d'eau, distillation ou extraction au moyen de solvants volatils notamment. Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage (expression à froid). L'huile essentielle est le plus souvent séparée de la phase aqueuse par un procédé physique n'entraînant pas de changement significatif de la composition.

Parmi les méthodes d'obtention d'une huile essentielle, on peut citer l'entraînement à la vapeur d'eau qui peut par exemple être réalisé par distillation sèche ou hydro-distillation.

L'hydro-distillation peut être réalisée sur un appareil en verre tel que celui défini dans la Pharmacopée Européenne pour la détermination de l'huile essentielle d'une matière végétale. L'entraînement à la vapeur correspond à la vaporisation en présence de vapeur d'eau d'une substance peu miscible à l'eau. La matière première est mise en présence d'eau portée à ébullition (hydro-distillation) ou de vapeur d'eau dans un alambic (distillation sèche). La vapeur d'eau entraîne la vapeur d'huile essentielle qui est condensée dans le réfrigérant pour être récupérée en phase liquide dans un vase florentin (ou essencier) où l'huile essentielle est séparée de l'eau par décantation. On appelle « eau aromatique » ou « hydrolat » ou « eau distillée florale », le distillat aqueux qui subsiste à l'entraînement à la vapeur d'eau, une fois la séparation de l'huile essentielle effectuée. Les huiles essentielles sont en général volatiles et liquides à température ambiante (25°C), ce qui les différencie des huiles dites fixes. Elles sont plus ou moins colorées et leur densité est en général inférieure à celle de l'eau. Elles sont liposolubles et solubles dans les solvants organiques usuels, entraînables à la vapeur d'eau et très peu solubles dans l'eau.

L'huile essentielle de géranium est extraite de plantes du genre *Pelargonium,* de la famille des Geraniacées. On utilise en particulier une huile essentielle extraite des fleurs de *Pelargonium graveolens.*

La menthe (du genre *Mentha*) est une plante de la famille des Lamiacées. On utilise en particulier l'huile

essentielle de menthe poivrée extraite de l'espèce *Mentha piperita,* L'huile essentielle provient de préférence des sommités de la plante.

Le lemongrass est une plante de la famille des Poacées (graminées). L'huile essentielle de lemongrass est extraite de l'espèce *Cymbopogon flexuosus.* Elle provient des parties herbacées de la plante, notamment des feuilles.

La mélisse est une plante du genre *Melissa* de la famille des Lamiacées. On utilise en particulier l'huile essentielle extraite de la plante *Melissa officinalis* de préférence à partir des parties aériennes et des feuilles de la plante.

Le romarin, ou *Rosmarinus officinalis,* est un arbrisseau de la famille des Lamiacées. On utilise en particulier l'huile essentielle extraite des parties aériennes, notamment des sommités fleuries de la plante.

Le lavandin (*Lavandula hybrida*) provient d'une hybridation de lavande. On utilise en particulier l'huile essentielle extraite des parties aériennes, notamment des sommités fleuries de la plante.

Il est entendu que l'invention comprend des mélanges des différentes huiles essentielles en toutes proportions.

Les proportions de chacune des huiles essentielles dans le mélange d'huile essentielles seront adaptées par l'homme du métier.

L'huile essentielle de géranium (*Pelargonium graveolens*) peut représenter de 0 à 50% du mélange d'huiles essentielles ; lorsqu'elle est présente dans le mélange, elle pourra représenter par exemple de 1 à 50 % p/v du mélange d'huile essentielle présent dans l'association selon l'invention, notamment de 5 à 45%, en particulier de 30 à 45%.

L'huile essentielle de menthe poivrée (*Mentha piperita*) peut représenter de 0 à 50% du mélange d'huiles essentielles ; lorsqu'elle est présente dans le mélange, elle pourra représenter par exemple de 1 à 50 % p/v du mélange d'huiles essentielles présent dans l'association selon l'invention, notamment de 5 à 40%, en particulier de 20 à 35%.

L'huile essentielle de lemongrass (*Cymbopogon tlexuosus*) peut représenter de 0 à 50% du mélange d'huiles essentielles ; lorsqu'elle est présente dans le mélange, elle pourra représenter par exemple de 1 à 50 % p/v du mélange d'huiles essentielles présent dans l'association selon l'invention, notamment de 5 à 30%, en particulier de 15 à 22%.

L'huile essentielle de mélisse officinale (*Melissa officinalis*) peut représenter de 0 à 50% du mélange d'huiles essentielles ; lorsqu'elle est présente dans le mélange, elle pourra représenter par exemple de 1 à 40 % p/v du mélange d'huiles essentielles présent dans l'association selon l'invention, notamment de 1 à 30%, en particulier de 2 à 15%.

L'huile essentielle de romarin (*Rosmarinus officinalis*) peut représenter de 0 à 50% du mélange d'huiles essentielles ; lorsqu'elle est présente dans le mélange, elle pourra représenter par exemple de 1 à 40 % p/v du mélange d'huiles essentielles présent dans l'association selon l'invention, notamment de 5 à 20%, en particulier de 10 à 17%.

L'huile essentielle de lavandin (*Lavandula hybrida*) peut représenter de 0 à 50% du mélange d'huiles essentielles ; lorsqu'elle est présente dans le mélange, elle pourra représenter par exemple de 1 à 40 % p/v du mélange d'huiles essentielles présent dans l'association selon l'invention, notamment de 2 à 20%, en particulier de 4 à 10%.

Un mélange d'huiles essentielles convenant à la mise en oeuvre de l'invention contient par exemple de 20 à 35 % d'huile essentielle de menthe poivrée, de 30 à 45% d'huile essentielle de géranium, de 2 à 15% d'huile essentielle de mélisse, de 15 à 22% d'huile essentielle de lemongrass, de 10 à 17% d'huile essentielle de romarin et de 4 à 10% d'huile essentielle de lavandin.

Dans toute la description, les expressions « compris entre...et ... » et « allant de ...à .. » doivent se comprendre bornes incluses, sauf si le contraire est spécifié.

Les utilisations et procédés selon l'invention sont, à moins qu'il ne soit stipulé différemment, de nature cosmétique, non-thérapeutique ; ils ont essentiellement une finalité esthétique.

Par prévenir ou prévention on entend le fait de réduire la probabilité de survenue ou de réduire la manifestation du phénomène concerné.

Par traiter, on entend le fait de supprimer, limiter ou retarder la manifestation du phénomène concerné.

L'association définie dans ce qui précède, ou la composition la contenant, sont notamment utiles pour traiter ou prévenir les désordres esthétiques cutanés qui sont des imperfections cutanées choisies parmi une peau luisante, une peau grasse, une peau présentant des orifices folliculaires ou des pores dilatés, une peau présentant des comédons et/ou points noirs, un grain de peau épais, une peau rugueuse ou présentant un relief irrégulier.

Une composition particulièrement adaptée à l'invention contient, dans un milieu physiologiquement, et notamment cosmétiquement acceptable, une association de (i) polyphénols extraits de vigne et/ou de l'un de ses dérivés et de (ii) un mélange d'huiles essentielles, tels que définis dans ce qui précède.

Dans une telle composition le ou les polyphénols ou dérivés de polyphénols sont présents à une concentration de 0,01 à 10%, en particulier de 0,02 à 10%; selon certains modes de réalisation la concentration en polyphénols et notamment en OPC extraits de raisin est inférieure à 1%, et notamment de 0,01 à 0,5% en poids par rapport au poids total de la composition.

Le mélange d'huiles essentielles choisies dans le groupe défini ci-dessus est présent dans les compositions selon l'invention à une concentration variant 0,02 à 5%, notamment de 0,02 à 1% en poids mais cette concentration pourra être supérieure ou égale à 0,04% en poids; en particulier la concentration du mélange d'huiles essentielles pourra varier de 0,04 à 0,5%, notamment de 0,04 à 0,4% en poids.

Les pourcentages sont exprimés en poids par rapport au poids total de la composition. L'invention concerne

ainsi une composition qui contient au moins :

(i) un polyphénol choisi parmi les monomères et oligomères de proanthocyanidines extraits de pépins de raisin, sous forme native ou sous forme estérifiée et leurs mélanges, et
(ii) un mélange d'huiles essentielles de menthe poivrée, de géranium, de mélisse officinale, de lemongrass, de romarin, et de lavandin.

Une composition selon l'invention contient en particulier au moins :

(i) un extrait de pépins de raisin contenant des polyphénols, stabilisé par estérification, l'extrait étant présent à une concentration de 0,01 à 10% en poids par rapport au poids total de la composition, et
(ii) un mélange d'huiles essentielles de menthe poivrée, de géranium, de mélisse officinale, de lemongrass, de romarin, et de lavandin, ledit mélange d'huiles essentielles étant présent à une concentration de 0,02 à 5% en poids par rapport au poids total de la composition

Dans des associations particulièrement adaptées à la mise en oeuvre de l'invention, le ratio massique polyphénols de raisin / huiles essentielles est compris entre 1:100 et 100:1. Avantageusement, le ratio massique polyphénols de raisin / huiles essentielles est compris entre 1:50 et 50:1, en particulier entre 1:25 et 25:1. Il peut notamment varier de 4:1 à 1 :4, et en particulier être de 1:1.
Les compositions selon l'invention sont de préférence des compositions cosmétiques, ou des compositions dermatologiques adaptées à une application sur la peau ou les phanères. La composition selon l'invention contient l'association polyphénols ou dérivés de polyphénols extraits de pépins de raisin et du mélange d'huiles essentielles telle que définie précédemment, et un milieu physiologiquement acceptable.
Par milieu physiologiquement acceptable on entend un milieu compatible avec la peau ou les phanères et ne provoquant pas d'effet indésirable lors de sa mise en contact avec les différentes parties du corps.
Il s'agit en particulier d'un milieu cosmétiquement acceptable. On entend ainsi un milieu ou des excipients bien tolérés par la peau et présentant une odeur et un aspect agréable.
Par produit ou composition cosmétique, on entend une substance ou un mélange destiné à être mis en contact avec les parties superficielles du corps humain ou avec les dents et les muqueuses buccales, en vue, exclusivement ou principalement, de les nettoyer, de les parfumer, d'en modifier l'aspect, de les protéger, de les maintenir en bon état ou de corriger les odeurs corporelles. Un procédé de traitement cosmétique au sens de l'invention a les mêmes objectifs.
Cette composition peut être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage et/ou produit d'hygiène pour la peau ou les phanères.
La composition selon l'invention peut se présenter sous toutes les formes galéniques normalement utilisées dans le domaine cosmétique, et elle peut être notamment sous forme d'une solution aqueuse ou huileuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.
Lorsque la composition utilisée selon l'invention est une émulsion, la proportion de la phase grasse peut aller de 0,5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co- émulsionnant sont présents, dans la composition, en une proportion allant de 0,1 à 30 % en poids, et de préférence de 0,5 à 20 % en poids par rapport au poids total de la composition.
Comme huiles utilisables dans l'invention, on peut citer les hydrocarbures d'origine minérale (huile minérale) ou synthétique (huile de vaseline, isohexadécane), les huiles d'origine végétale (huile d'amande d'abricot, de pépins de raisins, fraction liquide de beurre de karité, huile d'avocat, huile de soja), les huiles d'origine animale (lanoline), les huiles de synthèse (perhydrosqualène, tétraoctanoate de pentaérythrityle), les dérivés siliconés (cyclopentasiloxane, cyclohexasiloxane, dimethicone et polymères de silicone) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille), des beurres, des huiles hydrogénées.
Comme émulsionnants et co-émulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle, les esters de sucrose, les phospholipides.
De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les écrans, les pigments, les absorbeurs d'odeur, les chélatants, les alcools et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'association.

Comme gélifiants hydrophiles, on peut citer en particulier les polymères carboxyvinyliques (carbomères), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges, on peut citer par exemple, les particules de polyamide (Nylon) sous forme sphérique ou sous forme de microfibres ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

La composition utilisée selon l'invention peut en outre contenir au moins un actif additionnel. Cet actif additionnel peut en particulier être un actif additionnel de traitement des peaux grasses. L'actif additionnel peut notamment être un composé choisi parmi : les agents desquamants, les agents antimicrobiens, les agents apaisants, les agents anti-inflammatoires les agents anti-oxydants, les agents cicatrisants, les agents astringents, les agents hydratants, les agents favorisant la microcirculation cutanée ; les agents dépigmentants ; les agents antipollution et/ou anti-radicalaire, les filtres solaires, les agents modifiant la production de sébum, les agents rafraichissants et leurs mélanges.

L'agent desquamant peut notamment être choisi parmi les bêta-hydroxyacides comme l'acide salicylique ou un dérivé acylé d'acide salicylique, notamment un acide 5-acyl-salicylique tel que l'acide n-octanoyl-5-salicylique, l'acide n-dodécanoyl-5-salicylique et l'acide n-décanoyl-5-salicylique, sous forme libre ou salifiée, en particulier sous forme de sels obtenus par salification avec une base minérale ou organique. D'autres agents desquamants utilisables sont les alpha-hydroxyacides (AHA), tels que les acides glycolique, citrique, lactique, tartrique, malique ou mandélique, l'acide gentisique ou ses esters en particulier le gentisate de tocophérol, les oligofucoses, l'acide cinnamique, certains dérivés d'acide jasmonique, et/ou leurs dérivés et/ou leurs mélanges, des dérivés de sucre tels que l'O-octanoyl-6-D-maltose et la N-acétyl glucosamine.

En particulier, on peut utiliser l'acide salicylique.

Comme agents antioxydants, on peut citer notamment le tocophérol et ses esters, en particulier l'acétate de tocophérol ; l'acide ascorbique et ses dérivés, en particulier le phosphate d'ascorbyle de magnésium, l'ascorbyl glucoside et l'ascorbyl tetraisopalmitate ; l'acide férulique ; la sérine ; l'acide ellagique, la phlorétine, les chélatants, tels que le BHT, le BHA, le N,N'-bis(3,4,5-triméthoxybenzyl) éthylènediamine et ses sels, hydroxymatairesinol et leurs mélanges.

Avantageusement, la composition selon l'invention comprend au moins un actif additionnel choisi parmi les agents desquamants, les agents anti-oxydants et les agents apaisants.

En particulier, la composition cosmétique selon l'invention contient en outre au moins un actif choisi parmi la vitamine C et ses dérivés et la vitamine E ou ses dérivés tels que l'acétate de tocophérol.

D'autres actifs additionnels avantageusement contenus dans les compositions selon l'invention sont choisis parmi l'acide salicylique, la vitamine B3 et/ou l'hydrolat de rose, et leurs mélanges.

Les actifs additionnels pourront être présents dans la composition selon l'invention en une teneur allant de 0,001 % à 20% en poids par rapport au poids total de la composition, de préférence de 0,01% à 10%, encore plus préférentiellement de 0,5% à 5% et de préférence encore de 0,1% à 1% en poids par rapport au poids total de la composition.

L'invention a encore pour objet un procédé de traitement non thérapeutique cosmétique comprenant une étape d'application sur la peau ou les phanères de l'association ou des compositions selon l'invention.

Le procédé selon l'invention peut s'avérer tout particulièrement utile pour prévenir et/ou traiter les défauts esthétiques de la peau grasse ou à tendance grasse, en particulier pour traiter une peau présentant des orifices folliculaires ou des pores dilatés ou pour prévenir ce phénomène, en particulier pour réduire l'apparence et/ou la visibilité des pores, pour resserrer les pores et/ou diminuer la taille des pores, et/ou diminuer le nombre de pores visibles, pour prévenir et/ou traiter une peau présentant des orifices folliculaires ou des pores comblés de comédons, ou une peau présentant des comédons et/ou des points noirs.

Dans ce cadre, l'association pourra être présente dans des compositions contenant un milieu physiologiquement acceptable telles que définies plus haut.

Les compositions et associations selon l'invention pourront être appliquées directement sur la peau ou les phanères ou, de façon alternative, sur des supports cosmétiques ou dermatologiques de type occlusif ou non occlusif, destinés à être appliqués de façon localisée sur la peau, tels que les masques hydrogels, lingettes, tissus, ou biocellulose.
L'application pourra par exemple être quotidienne, pluriquotidienne ou hebdomadaire. Elle pourra être poursuivie pendant plusieurs jours et/ou plusieurs semaines, voire plus longtemps ; elle pourra être continue ou bien par exemple poursuivie pendant 1 à 2 mois puis reprise après une interruption.
La composition cosmétique peut être rincée ou non après avoir été appliquée sur la peau. Par ailleurs, après l'application de la composition cosmétique selon l'invention, on peut appliquer sur la surface de la peau, une composition comprenant un ou plusieurs actifs choisis parmi les antibactériens, les antifongiques et/ou des poudres.
L'invention concerne aussi l'utilisation d'une association de (i) polyphénol choisi parmi les monomères et oligomères de flavonoïdes extraits de pépins de raisin, leurs dérivés et leurs mélanges, et (ii) d'huiles essentielles de menthe poivrée, de géranium, de mélisse officinale, de lemongrass, de romarin et de lavandin tels que définis dans le présent texte, pour la préparation d'une composition, notamment cosmétique. La composition est adaptée a une application sur la peau ou les phanères.
L'invention concerne encore un procédé de préparation d'une composition cosmétique comprenant une étape dans laquelle on mélange une quantité efficace de (i) polyphénol choisi parmi les monomères et oligomères de flavonoïdes extraits de pépins de raisin, leurs dérivés et leurs mélanges, et (ii) d'huiles essentielles de menthe poivrée, de géranium, de mélisse officinale, de lemongrass, de romarin et de lavandin tels que définis dans le présent texte, et optionnellement un excipient physiologiquement acceptable.
D'autres caractéristiques et avantages de l'invention sont illustrés dans les exemples qui suivent.
Dans ces exemples on se réfèrera à la figure unique en annexe. Cette figure est l'isobologramme représentant l'effet synergique d'un extrait de pépins de raisin en association avec un mix de 6 huiles essentielles.

**Exemple 1 : activité sur *P.* acnes dans une utilisation thérapeutique hors invention**

On évalue l'activité antimicrobienne de deux actifs, respectivement :

- un extrait de pépins de raisin
- un mélange d'huiles essentielles de menthe poivrée, de géranium, de mélisse officinale, de lemongrass, de romarin, de lavandin

L'extrait de pépin de raisins est dosé à 97% d'oligomères de proanthocyanidines (OPC).
L'activité antimicrobienne a été testée sur la bactérie *Propionibacterium acnes* ATCC6919, souche de référence sensible aux antibiotiques.
Les bactéries ont été cultivées dans un milieu nutritif liquide BHI (Brain Heart Infusion) ; les cultures ont été incubées en anaérobiose à 36°C pendant 3 à 5 jours.
Afin d'évaluer l'activité antimicrobienne des ingrédients pris individuellement, un test de Concentration Minimale Inhibitrice (CMI) a été effectué. La mesure de CMI est une méthode internationale de référence pour quantifier une activité antimicrobienne en milieu liquide. Le test a été effectué en microplaques de 96 puits. 100ul de produit à tester (à concentration double de la concentration à tester) sont mis en contact avec 100ul de bouillon nutritif (à concentration double) contenant la bactérie à une densité de 2-6.$10^5$ ufc/ml.
Après incubation de la microplaque pendant 3-5 jours, la croissance bactérienne est évaluée par mesure de densité optique à 620 nm. Les résultats sont exprimés en pourcentage de croissance calculé par rapport à un témoin de croissance, selon l'équation suivante :

$$Pourcentage\ de\ croissance = \frac{DO\ mesurée\ produit\ testé - DO\ témoin\ d'absorbance}{DO\ témoin\ de\ croissance}$$

où : témoin d'absorbance = 100µl milieu nutritif sans bactéries + 100µl produit à tester témoin de croissance = 100µl milieu nutritif avec bactéries + solvant de dilution des produits à tester
Est considérée comme inhibitrice la première concentration en produit testée permettant l'obtention d'un pourcentage de croissance inférieur ou égal à 20%.
Une activité antimicrobienne sur la croissance de *P. acnes* a été observée avec les 2 composés testés. Les CMI mesurées sont :

- CMI (extrait de pépins de raisin) = 0,05 % (m/v)

- CMI (mix de 6 huiles essentielles) = 0,1 % (m/v)

L'évaluation de l'activité de l'association a été effectuée selon le même protocole expérimental et en utilisant la méthode de l'échiquier pour le choix des concentrations à tester (CMI/4 - CMI/2 - CMI - 2CMI - 4 CMI pour chacun des deux composés). Afin d'évaluer une éventuelle synergie entre les deux composés, un calcul de Fraction de Concentration Inhibitrice (FIC) a été effectué :

$$\text{FIC index} = \text{FIC}_A + \text{FIC}_B$$

Avec :

$$\text{FIC}_A = \text{CMI}_{\text{A avec B}} / \text{CMI}_{\text{A}}$$

$$\text{FIC}_B = \text{CMI}_{\text{B avec A}} / \text{CMI}_{\text{B}}$$

$\text{CMI}_{\text{A avec B}}$ : Concentration en produit A dans l'association permettant d'obtenir l'effet inhibiteur
$\text{CMI}_{\text{B avec A}}$ : Concentration en produit B dans l'association permettant d'obtenir l'effet inhibiteur
$\text{CMI}_{\text{A}}$ : CMI du produit A seul
$\text{CMI}_{\text{B}}$ : CMI du produit B seul

Selon cette méthode, si :

| | |
|---|---|
| - FIC index $\leq$ 0,75 | on conclut en un effet synergique des 2 composés |
| - FIC index = 1 | on conclut en un effet additif des 2 composés |
| - 1 $\leq$ FIC index < 2 | on conclut en une indifférence de l'association des 2 composés |
| - FIC index $\geq$ 2 | on conclut en un antagonisme des 2 composés |

Au ratio d'extrait de pépins de raisin : mix d'huiles essentielles de 1 :1 on démontre :
$\text{FIC}_A$ = 0,5 et $\text{FIC}_B$ = 0,25

**FIC index (extrait de pépins de raisin + mix de 6 huiles essentielles) = 0,75**

Au ratio d'extrait de pépins de raisin : mix d'huiles essentielles de 1 :4 on démontre :
$\text{FIC}_A$ = 0,25 et $\text{FIC}_B$ = 0,5

**FIC index (extrait de pépins de raisin + mix de 6 huiles essentielles) = 0,75**

Ce résultat prouve donc une interaction synergique.

Un isobologramme représentatif est représenté en figure en annexe. Le profil de l'isobologramme est différent selon le type d'interaction entre les ingrédients. La ligne en pointillé indique les valeurs de FIC index égales à 1 qui indiquent une interaction purement indifférente. Un isobologramme concave met en évidence que la réponse de l'association des deux ingrédients est supérieure à la somme de leur réponse individuelle, ce qui correspond à une interaction synergique. Un isobologramme convexe met en évidence que la réponse de l'association des deux ingrédients est inférieure à la somme de leur réponse individuelle, ce qui correspond à une interaction antagoniste.

La figure démontre une activité antimicrobienne synergique de la combinaison de l'extrait de pépins de raisin et du mix d'huiles essentielles contre P. *acnes*.

**Exemple 2 : compositions selon l'invention:**

Lotion

| Nom Commun | % |
|---|---|
| Eau | QSP 100 |
| Glycérine | 5,00 |
| Extraits de Potyphénols de vigne | 0,01-1% |
| Mélange d'huiles essentielles | 0,02-1% |
| Alcool | 10,00 |

On mélange les différents constituants de la composition pour obtenir une lotion. Celle-ci sera appliquée le matin et/ou le soir sur l'ensemble du visage et du cou en évitant le contour des yeux.

Gel Crème

| Nom Commun | % |
|---|---|
| Eau | QSP 100 |
| Butylène glycol | 2,00 |
| C10/30 alkyl acrylates crosspolymer | 0,25 |
| Gomme xanthane | 0,15 |
| Hexyldécanol | 3,00 |
| Acide salicylique | 0,1 |
| Sodium hyaluronate | 0,2 |
| Extraits de Polyphénols de vigne | 0,01-1% |
| Mélange d'huiles essentielles | 0,02-1% |
| Alcool | 5,00 |

Le gel-crème sera appliqué le matin et/ou le soir sur l'ensemble du visage et du cou en évitant le contour des yeux.

Emulsion

| Nom Commun | % |
|---|---|
| Eau | QSP 1 00 |
| Gomme de xanthane | 0,30 |
| Acide phytique | 0,05 |
| Acide salicylique | 0,1 |
| Coco-caprylate/caprale | 3,00 |
| Gaprylic/Capric triglyceride | 5,0 |
| Mélange de C14-22 Alcohol et C12-20 alkyl glucoside | 3,00 |
| Extrait de Polyphénols de vigne | 0,01-1% |
| Mélange d'huiles essentielles | 0,02-1% |
| poudre matifiante | 2,0 |
| Alcool | 5 |

L'émulsion sera appliquée le matin et/ou le soir sur l'ensemble du visage et du cou en évitant le contour des yeux.

**Revendications**

1. Utilisation non thérapeutique cosmétique d'une association de

(i) au moins un polyphénol choisi parmi les monomères et oligomères de flavonoïdes, leurs dérivés et leurs mélanges, les polyphénols étant extraits de pépins de raisin,
les unités monomères de dérivés de flavonoïde répondant à la structure (I) suivante,

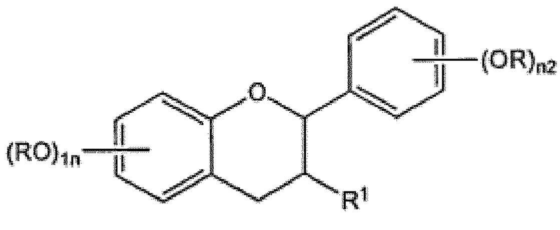

(I)

dans laquelle

- R1 représente un groupe -OR2, un atome d'hydrogène, ou un substituant R3 ; R2 et R3 identiques ou différents, étant tels que défini ci-dessous pour R4,
- au moins la majorité des substituants R représentent un groupe -COR4, R4 étant un radical alkyle d'au moins deux atomes de carbone, linéaire ou ramifié, saturé ou insaturé, un radical aryle, arylalkyle ou arylalkylène,
- le ou les autres substituants R qui ne représentent pas un groupe -COR4 étant un atome d'hydrogène, ou un groupe alkyle, et
- n1 et n2, identiques ou différents l'un de l'autre, sont des nombres de 1 à 3, correspondant au nombre de substitutions sur un cycle,

les motifs monomères étant reliés dans les oligomères par des liaisons carbone-carbone et /ou un pont éther entre les unités, et
(ii) d'huiles essentielles de menthe poivrée, de géranium, de mélisse officinale, de lemongrass, de romarin, et de lavandin,

pour traiter et/ou prévenir les peaux grasses ou à tendance grasse et/ou les désordres esthétiques cutanés qui leur sont associés.

2. Utilisation non thérapeutique cosmétique selon la revendication 1, **caractérisée en ce que** l'association contient au moins un polyphénol choisi parmi les monomères et oligomères de flavan-3-ols, tels que les monomères et oligomères de proanthocyanidines (OPC), des esters de ceux-ci et leurs mélanges.

3. Utilisation non thérapeutique cosmétique selon l'une quelconque des revendications précédentes, **caractérisée en ce que** l'association est utilisée dans une composition cosmétique comprenant un milieu physiologiquement acceptable.

4. Utilisation non thérapeutique cosmétique selon l'une quelconque des revendications précédentes, **caractérisée en ce que** l'association ou la composition cosmétique la contenant sont utilisées pour traiter ou prévenir les désordres esthétiques cutanés qui sont des imperfections cutanées choisies parmi une peau luisante, une peau grasse, une peau présentant des orifices folliculaires ou des pores dilatés, une peau présentant des comédons et/ou points noirs, un grain de peau épais, une peau rugueuse ou présentant un relief irrégulier.

5. Procédé de traitement non thérapeutique cosmétique des peaux grasses ou à tendance grasse et/ou des désordres esthétiques cutanés associés aux peaux grasses ou à tendance grasse, **caractérisé en ce qu'**il comprend l'application topique sur la peau d'une association de

(i) au moins un polyphénol extrait de pépins de raisin, et
(ii) d'huiles essentielles de menthe poivrée, de géranium, de mélisse officinale, de lemongrass, de romarin et

de lavandin,

ou d'une composition cosmétique contenant ladite association telle que définie dans l'une des revendications 1 à 3.

**6.** Procédé de traitement non thérapeutique cosmétique selon la revendication 5, **caractérisé en ce qu'**il comprend l'application topique sur la peau d'une composition contenant de 0,01% à 10% en poids de polyphénols et de 0,02% à 5% en poids d'huiles essentielles, dans un milieu physiologiquement acceptable.

**7.** Composition cosmétique contenant une association de

(i) au moins un polyphénol extrait de pépins de raisin choisi parmi les monomères et oligomères de flavonoïdes, leurs dérivés et leurs mélanges, les polyphénols étant présents à raison de de 0,01 % à 10% en poids,
les unités monomères de dérivés de flavonoïde répondant à la structure (I) suivante,

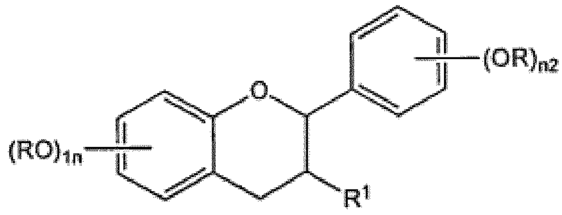

(I)

dans laquelle

- R1 représente un groupe -OR2, un atome d'hydrogène, ou un substituant R3 ; R2 et R3 identiques ou différents, étant tels que défini ci-dessous pour R4,
- au moins la majorité des substituants R représentent un groupe -COR4, R4 étant un radical alkyle d'au moins deux atomes de carbone, linéaire ou ramifié, saturé ou insaturé, un radical aryle, arylalkyle ou arylalkylène,
- le ou les autres substituants R qui ne représentent pas un groupe -COR4 étant un atome d'hydrogène, ou un groupe alkyle, et
- n1 et n2, identiques ou différents l'un de l'autre, sont des nombres de 1 à 3, correspondant au nombre de substitutions sur un cycle,

les motifs monomères étant reliés dans les oligomères par des liaisons carbone-carbone et /ou un pont éther entre les unités, et
(ii) d'huiles essentielles de menthe poivrée, de géranium, de mélisse officinale, de lemongrass, de romarin et de lavandin, les huiles essentielles étant présentes à une concentration 0,02 à 5% en poids,

tels que définis dans l'une quelconque des revendications 1 à 4, dans un milieu physiologiquement acceptable.

**8.** Composition selon la revendication 7, **caractérisée en ce qu'**elle contient en outre au moins un actif additionnel choisi parmi les agents desquamants et les agents anti-oxydants et les agents apaisants.

**9.** Composition cosmétique selon l'une au moins des revendications 7 ou 8, **caractérisée en ce qu'**elle contient au moins

(i) un polyphénol choisi parmi les monomères et oligomères de proanthocyanidines extraits de pépins de raisin, sous forme native ou sous forme estérifiée et leurs mélanges, et
(ii) un mélange d'huiles essentielles de menthe poivrée, de géranium, de mélisse officinale, de lemongrass, de romarin, et de lavandin.

**10.** Composition cosmétique selon l'une au moins des revendications 7 à 9, **caractérisée en ce qu'**elle contient au moins

(i) un extrait de pépins de raisin contenant des polyphénols, stabilisé par estérification, l'extrait étant présent à une concentration de 0,01 à 10% en poids par rapport au poids total de la composition, et

(ii) un mélange d'huiles essentielles de menthe poivrée, de géranium, de mélisse officinale, de lemongrass, de romarin, et de lavandin, ledit mélange d'huiles essentielles étant présent à une concentration de 0,01 à 10% en poids par rapport au poids total de la composition.

**11.** Composition cosmétique selon l'une quelconque des revendications 7 à 10, **caractérisée en ce que** le ratio massique polyphénols de raisin / huiles essentielles est compris entre 1:100 et 100:1.

## Patentansprüche

**1.** Nicht-therapeutische kosmetische Verwendung einer Verbindung von

(i) mindestens einem Polyphenol, das aus Monomeren und Oligomeren von Flavonoiden, deren Derivaten und deren Gemischen ausgewählt ist, wobei die Polyphenole Extrakte von Traubenkernen sind,
wobei die monomeren Einheiten von Flavonoidderivaten der folgenden Struktur (I) entsprechen,

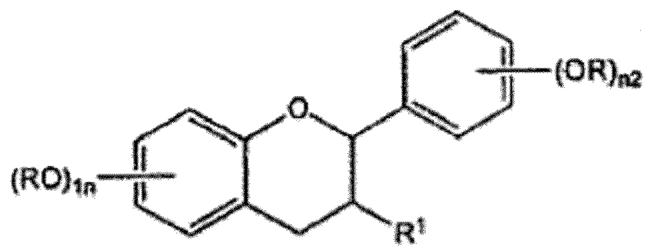

(I)

in der

- R1 für eine -OR2-Gruppe, ein Wasserstoffatom oder einen R3-Substituenten steht; R2 und R3, die gleich oder verschieden sind, wie im Folgenden für R4 definiert sind,
- mindestens die Mehrheit von R-Substituenten für eine -COR4-Gruppe steht, wobei R4 ein Alkylrest mit mindestens zwei Kohlenstoffatomen, linear oder verzweigt, gesättigt oder ungesättigt, ein Arylrest, Arylalkyl oder Arylalkylen ist,
- der oder die anderen R-Substituenten, die nicht für eine -COR4-Gruppe stehen, ein Wasserstoffatom oder eine Alkylgruppe sind und
- n1 und n2, die gleich oder voneinander verschieden sind, Zahlen von 1 bis 3 sind, der Anzahl von Substitutionen an einem Zyklus entsprechend, wobei die monomeren Motive in den Oligomeren durch Kohlenstoff-Kohlenstoff-Bindungen und/oder eine Etherbrücke zwischen den Einheiten verbunden sind, und

(ii) ätherischen Ölen von Pfefferminze, Geranie, Melisse, Zitronengras, Rosmarin und Lavandin,

zur Behandlung und/oder Vorbeugung von fettiger Haut oder zu Fett neigender Haut und/oder ästhetischen Hautstörungen, die damit assoziiert sind.

**2.** Nicht-therapeutische kosmetische Verwendung nach Anspruch 1, **dadurch gekennzeichnet, dass** die Verbindung mindestens ein Polyphenol enthält, das aus Monomeren und Oligomeren von Flavan-3-olen, wie Monomeren und Oligomeren von Proanthocyanidinen (OPC), Estern dieser und deren Gemischen, ausgewählt ist.

**3.** Nicht-therapeutische kosmetische Verwendung nach einem der vorhergehenden Ansprüche, **dadurch gekennzeichnet, dass** die Verbindung in einer kosmetischen Zusammensetzung verwendet wird, die ein physiologisch annehmbares Medium umfasst.

**4.** Nicht-therapeutische kosmetische Verwendung nach einem der vorhergehenden Ansprüche, **dadurch gekennzeichnet, dass** die Verbindung oder die kosmetische Zusammensetzung, die diese enthält, zur Behandlung oder Vorbeugung von ästhetischen Hautstörungen verwendet wird, die Hautmakel sind, die aus einer glänzenden Haut, einer fettigen Haut, einer Haut, die Follikelöffnungen oder vergrößerte Poren aufweist, einer Haut, die Komedonen

und/oder Mitesser aufweist, einer dicken Hautstruktur, einer rauen Haut oder einer unebenmäßigen Haut ausgewählt sind.

5. Verfahren zur nicht-therapeutischen kosmetischen Behandlung von fettiger Haut oder zu Fett neigender Haut und/oder ästhetischen Hautstörungen, die mit fettiger Haut oder zu Fett neigender Haut assoziiert sind, **dadurch gekennzeichnet, dass** es die topische Anwendung einer Verbindung von

(i) mindestens einem Polyphenol, das aus Traubenkernen extrahiert wurde, und
(ii) ätherischen Ölen von Pfefferminze, Geranie, Melisse, Zitronengras, Rosmarin und Lavandin,

oder einer kosmetischen Zusammensetzung, die die wie in einem der Ansprüche 1 bis 3 definierte Verbindung enthält, auf die Haut umfasst.

6. Verfahren zur nicht-therapeutischen kosmetischen Behandlung nach Anspruch 5, **dadurch gekennzeichnet, dass** es die topische Anwendung einer Zusammensetzung, die von 0,01 Gew.-% bis 10 Gew.-% Polyphenole und von 0,02 Gew.-% bis 5 Gew.-% ätherische Öle enthält, in einem physiologisch annehmbaren Medium auf die Haut umfasst.

7. Kosmetische Zusammensetzung, enthaltend eine Verbindung von

(i) mindestens einem Polyphenol, das aus Traubenkernen extrahiert wurde und aus Monomeren und Oligomeren von Flavonoiden, deren Derivaten und deren Gemischen ausgewählt ist, wobei die Polyphenole im Umfang von 0,01 Gew.-% bis 10 Gew.-% vorliegen,
wobei die monomeren Einheiten von Flavonoidderivaten der folgenden Struktur (I) entsprechen,

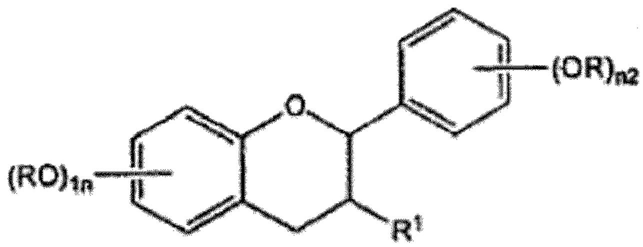

(I)

in der

- R1 für eine -OR2-Gruppe, ein Wasserstoffatom oder einen R3-Substituenten steht; R2 und R3, die gleich oder verschieden sind, wie im Folgenden für R4 definiert sind,
- mindestens die Mehrheit von R-Substituenten für eine -COR4-Gruppe steht, wobei R4 ein Alkylrest mit mindestens zwei Kohlenstoffatomen, linear oder verzweigt, gesättigt oder ungesättigt, ein Arylrest, Arylalkyl oder Arylalkylen ist,
- der oder die anderen R-Substituenten, die nicht für eine -COR4-Gruppe stehen, ein Wasserstoffatom oder eine Alkylgruppe sind und
- n1 und n2, die gleich oder voneinander verschieden sind, Zahlen von 1 bis 3 sind, der Anzahl von Substitutionen an einem Zyklus entsprechend,

wobei die monomeren Motive in den Oligomeren durch Kohlenstoff-Kohlenstoff-Bindungen und/oder eine Etherbrücke zwischen den Einheiten verbunden sind, und
(ii) ätherischen Ölen von Pfefferminze, Geranie, Melisse, Zitronengras, Rosmarin und Lavandin, wobei die ätherischen Öle in einer Konzentration von 0,02 bis 5 Gew.-% vorliegen,

wie in einem der Ansprüche 1 bis 4 definiert, in einem physiologisch annehmbaren Medium.

8. Zusammensetzung nach Anspruch 7, **dadurch gekennzeichnet, dass** sie weiterhin mindestens einen zusätzlichen Wirkstoff enthält, der aus Peeling-Mitteln und Antioxidationsmitteln und beruhigenden Mitteln ausgewählt ist.

**9.** Kosmetische Zusammensetzung nach einem von mindestens den Ansprüchen 7 oder 8, **dadurch gekennzeichnet, dass** sie mindestens

(i) ein Polyphenol, das aus Monomeren und Oligomeren von Proanthocyanidinen, die aus Traubenkernen extrahiert wurden, in nativer Form oder in veresterter Form oder deren Gemischen ausgewählt ist, und
(ii) ein Gemisch von ätherischen Ölen von Pfefferminze, Geranie, Melisse, Zitronengras, Rosmarin und Lavandin,

enthält.

**10.** Kosmetische Zusammensetzung nach einem von mindestens den Ansprüchen 7 bis 9, **dadurch gekennzeichnet, dass** sie mindestens

(i) einen Extrakt von Traubenkernen, der Polyphenole enthält, durch Veresterung stabilisiert, wobei der Extrakt in einer Konzentration von 0,01 bis 10 Gew.-% vorliegt, bezogen auf das Gesamtgewicht der Zusammensetzung, und
(ii) ein Gemisch von ätherischen Ölen von Pfefferminze, Geranie, Melisse, Zitronengras, Rosmarin und Lavandin, wobei das Gemisch von ätherischen Ölen in einer Konzentration von 0,01 bis 10 Gew.-% vorliegt, bezogen auf das Gesamtgewicht der Zusammensetzung,

enthält.

**11.** Kosmetische Zusammensetzung nach einem der Ansprüche 7 bis 10, **dadurch gekennzeichnet, dass** das Massenverhältnis von Traubenpolyphenolen / ätherischen Ölen zwischen 1:100 und 100:1 liegt.

**Claims**

**1.** A non-therapeutic cosmetic use of a combination of

(i) at least one polyphenol selected from flavonoid monomers and oligomers, derivatives thereof and mixtures thereof, the polyphenols being extracted from grape seeds,
the monomer units of flavonoid derivatives corresponding to the following structure (I):

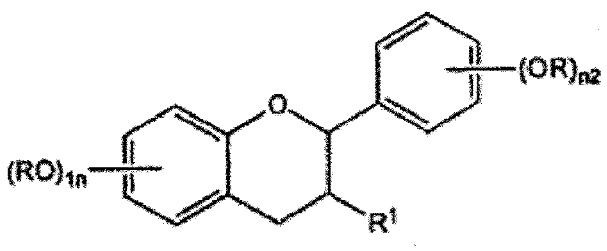

(I)

wherein

- R1 represents a -OR2 group, a hydrogen atom, or a substituent R3; R2 and R3 identical or different, being as defined below for R4,
- at least most of the substituents R represent a -COR4 group, R4 being an alkyl radical of at least two carbon atoms, linear or branched, saturated or unsaturated, an aryl, arylalkyl or arylalkylene radical,
- the other substituent(s) R which does/do not represent a -COR4 group being a hydrogen atom, or an alkyl group, and
- n1 and n2, identical or different from each other, are numbers from 1 to 3, corresponding to the number of substitutions on a cycle,

the monomer units being bonded in the oligomers by carbon-carbon bonds and/or an ether bridge between the units, and
(ii) essential oils of peppermint, geranium, lemon balm, lemongrass, rosemary, and lavandin,

for treating and/or preventing oily or oily-prone skins and/or aesthetic skin disorders associated thereto.

2. The non-therapeutic cosmetic use according to claim 1, **characterized in that** the combination contains at least one polyphenol selected from flavan-3-ol monomers and oligomers, such as proanthocyanidin monomers and oligomers (OPC), esters of these and mixtures thereof.

3. The non-therapeutic cosmetic use according to any one of the preceding claims, **characterized in that** the combination is used in a cosmetic composition comprising a physiologically acceptable medium.

4. The non-therapeutic cosmetic use according to any one of the preceding claims, **characterized in that** the combination or the cosmetic composition containing the same are used to treat or prevent aesthetic skin disorders which are skin imperfections selected from a glossy skin, an oily skin, a skin with follicular orifices or dilated pores, a skin with comedones and/or blackheads, a thick skin texture, a rough skin or with an irregular relief.

5. A method for non-therapeutic cosmetic treatment of oily or oily-prone skins and/or aesthetic skin disorders associated with oily or oily-prone skins, **characterized in that** it comprises the topical application to the skin of a combination of

(i) at least one polyphenol extracted from grape seeds, and
(ii) essential oils of peppermint, geranium, lemon balm, lemongrass, rosemary and lavandin,

or of a cosmetic composition containing said combination as defined in any of claims 1 to 3.

6. The non-therapeutic cosmetic treatment method according to claim 5, **characterized in that** it comprises the topical application to the skin of a composition containing from 0.01% to 10% by weight of polyphenols and from 0.02% to 5% by weight of essential oils, in a physiologically acceptable medium.

7. A cosmetic composition containing a combination of

(i) at least one polyphenol extracted from grape seeds selected from flavonoid monomers and oligomers, derivatives thereof and mixtures thereof, the polyphenols being present in an amount from 0.01% to 10% by weight, the monomer units of flavonoid derivatives corresponding to the following structure (I):

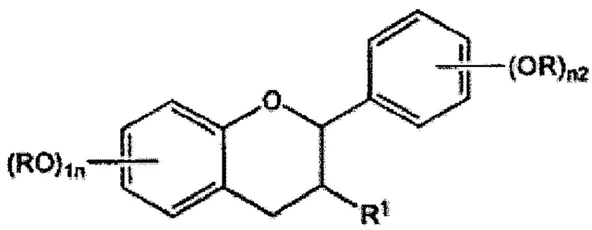

(I)

wherein

- R1 represents a -OR2 group, a hydrogen atom, or a substituent R3; R2 and R3 identical or different, being as defined below for R4,
- at least most of the substituents R represent a -COR4 group, R4 being an alkyl radical of at least two carbon atoms, linear or branched, saturated or unsaturated, an aryl, arylalkyl or arylalkylene radical,
- the other substituent(s) R which does/do not represent a -COR4 group being a hydrogen atom, or an alkyl group, and
- n1 and n2, identical or different from each other, are numbers from 1 to 3, corresponding to the number of substitutions on a cycle,

the monomer units being bonded in the oligomers by carbon-carbon bonds and/or an ether bridge between the units, and
(ii) essential oils of peppermint, geranium, lemon balm, lemongrass, rosemary and lavandin, the essential oils being present at a concentration of 0.02 to 5% by weight,

as defined in any one of claims 1 to 4, in a physiologically acceptable medium.

**8.** The composition according to claim 7, **characterized in that** it further contains at least one additional active agent selected from desquamating agents and antioxidants and soothing agents.

**9.** The cosmetic composition according to at least one of claims 7 or 8, **characterized in that** it contains at least

(i) one polyphenol selected from the proanthocyanidin monomers and oligomers extracted from grape seeds, in native form or in esterified form and mixtures thereof, and
(ii) a mixture of essential oils of peppermint, geranium, lemon balm, lemongrass, rosemary, and lavandin.

**10.** The cosmetic composition according to at least one of claims 7 to 9, **characterized in that** it contains at least

(i) a grape seed extract containing polyphenols, stabilized by esterification, the extract being present at a concentration of 0.01 to 10% by weight with respect to the total weight of the composition, and
(ii) a mixture of essential oils of peppermint, geranium, lemon balm, lemongrass, rosemary, and lavandin, said mixture of essential oils being present at a concentration of 0.01 to 10% by weight with respect to the total weight of the composition.

**11.** The cosmetic composition according to any one of claims 7 to 10, **characterized in that** the mass ratio of grape polyphenols/essential oils is comprised between 1:100 and 100:1.

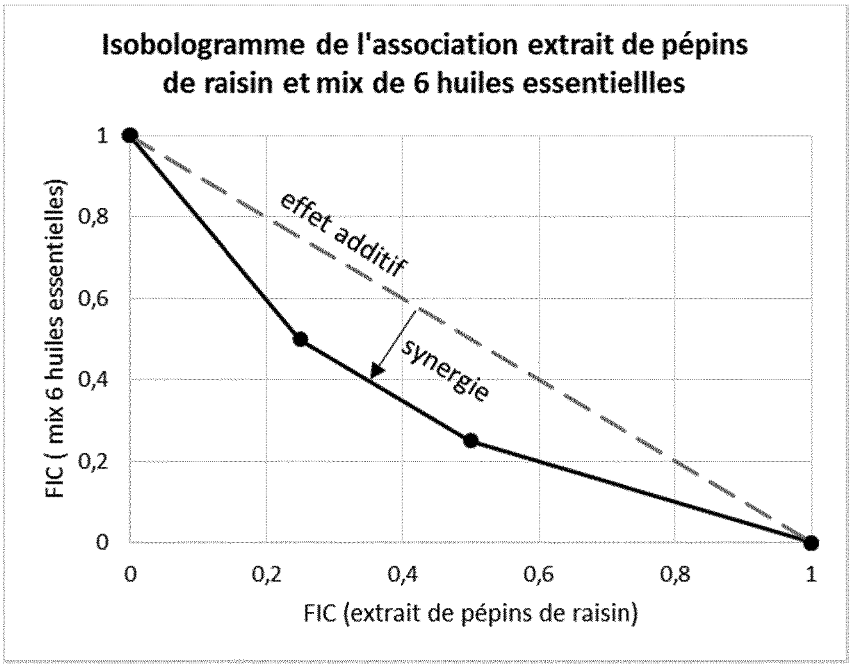
Isobologramme de l'association extrait de pépins de raisin et mix de 6 huiles essentiellles
FIC ( mix 6 huiles essentielles)
1
0,8
0,6
0,4
0,2
0
0
0,2
0,4
0,6
0,8
1
FIC (extrait de pépins de raisin)
effet additif
synergie

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 3006890
- FR 3004937
- FR 3004939
- CN 104146915
- WO 2011128714 A